# FASCICULE DE BREVET EUROPEEN

(11) **EP 4 402 117 B1**
(45) Date de publication et mention de la délivrance du brevet: **29.10.2025**
(21) Numéro de dépôt: 22789951.5
(22) Date de dépôt: 08.09.2022
(51) Int. Cl.: C07C 7/00, C07C 7/04, C07C 15/46, C07C 15/073, B01D 3/14, C08J 11/12, C10G 1/10, B01D 3/32, C10G 7/00, C10G 1/00

(54) **PROCÉDÉ DE PURIFICATION D'UNE CHARGE ISSUE D'UN PROCÉDÉ DE DÉPOLYMÉRISATION DE COMPOSÉS STYRÉNIQUES**
VERFAHREN ZUR REINIGUNG EINES EINSATZSTOFFS AUS EINEM VERFAHREN ZUR DEPOLYMERISATION VON STYROLVERBINDUNGEN
METHOD FOR PURIFYING A FEEDSTOCK RESULTING FROM A METHOD FOR DEPOLYMERISING STYRENE COMPOUNDS

(30) Priorité: 16.09.2021 FR 2109745
(43) Date de publication de la demande: 24.07.2024
(73) Titulaire: COMPAGNIE GENERALE DES ETABLISSEMENTS MICHELIN, 63000 Clermont-Ferrand (FR)
(72) Inventeur: PACHECO, Nuno, 63040 Clermont-Ferrand Cedex 9 (FR); KIENER, Pierre, 63040 Clermont-Ferrand Cedex 9 (FR); SÉRÉ-PEYRIGAIN, Pierre, 63040 Clermont-Ferrand Cedex 9 (FR)
(74) Mandataire: M.F.P. Michelin
(86) Numéro de dépôt international: PCT/FR2022/051700
(87) Numéro de publication internationale: WO 2023/041862

(56) Documents cités:
- WO-A1-2014/098816
- WO-A1-2020/144165

## Description

### Domaine technique de l'invention

La présente invention est relative au domaine des procédés de séparation du styrène.

### Art antérieur

Le styrène est un monomère très largement utilisé dans l'industrie, que ce soit par exemple pour la production de polystyrène, dont les domaines d'application sont multiples, ou la production d'élastomères tels que le caoutchouc styrène-butadiène (SBR). Il peut être obtenu de multiples façons, la principale étant par la déshydrogénation de l'éthylbenzène, ou dans une moindre mesure par l'oxydation de l'éthylbenzène suivie de la réaction sur le propylène puis de la déshydratation du produit obtenu.

Dans une optique de réduction de pression sur les ressources fossiles, des développements récents ont porté sur la dépolymérisation de composés styréniques.

Quelle que soit la méthode utilisée, le styrène produit doit être purifié afin d'atteindre les spécifications requises par les procédés de polymérisation. Les puretés requises, typiquement plus de 99,8% poids en styrène, conduisent à mettre en œuvre des procédés de séparation conséquents, en termes de nombre d'étapes et de consommation énergétique.

Le document US 2,457,361 décrit un procédé permettant de séparer le styrène d'une charge issue de la production de styrène par déshydrogénation de l'éthylbenzène, en séparant dans un premier temps le benzène et le toluène, puis les composés plus lourds que le styrène, et enfin l'éthylbenzène par un enchaînement de sept colonnes de distillation dont la plupart sont opérées sous vide afin de limiter l'exposition à la température du styrène. Cet enchaînement complexe est principalement la conséquence de la forte teneur en éthylbenzène de la charge à traiter.

Les charges issues de procédés de dépolymérisation de composés styréniques présentent des teneurs en éthylbenzène plus faibles que les procédés « classiques » dans lesquels le styrène est un produit de réaction de l'éthylbenzène. Toutefois, ces charges comprennent une teneur plus importante en composés dits « lourds », c'est-à-dire dont la température d'ébullition est supérieure à celle du styrène, et nécessitent d'être refroidies rapidement afin d'éviter la polymérisation du styrène.

Le document WO 2020/144165 décrit la séparation du styrène d'une charge issue de la pyrolyse de résidus styréniques en procédant dans un premier temps à la séparation des composés lourds, puis la séparation de l'éthylbenzène, et enfin la séparation des composés inhibiteurs de polymérisation. La charge est refroidie rapidement au niveau du plateau d'alimentation de la première colonne de distillation. Ce document enseigne que les composés « lourds » sont particulièrement visqueux et nécessitent d'être séparés tôt dans le procédé de purification du styrène. Ces caractéristiques des composés lourds nécessitent une conception particulière des plateaux de séparation des colonnes à distiller et de leurs rebouilleurs.

Poursuivant ses recherches, la demanderesse a découvert un enchainement d'étapes permettant de traiter une charge issue d'un procédé de dépolymérisation de composés styréniques afin d'obtenir un flux comprenant majoritairement du styrène aux spécifications nécessaires pour alimenter un procédé de polymérisation, avec en particulier des teneurs très faibles en composés « lourds », dont par exemple l'alpha-méthyl-styrène.

### Description détaillée de l'invention

L'invention concerne un procédé de purification d'une charge issue d'un procédé de dépolymérisation de composés styréniques, nommée charge du procédé de purification, comprenant au moins les étapes suivantes :
a. Une étape de séparation mettant en œuvre une colonne de distillation alimentée en fond de colonne par la charge du procédé de purification et produisant en tête de colonne un extrait riche en composés léger, en fond un raffinat riche en composés lourds, et par un soutirage latérale un flux riche en styrène, ladite colonne ayant pour seul apport de chaleur ladite charge du procédé de purification ;
b. Une étape de séparation du flux riche en styrène en au moins un flux comprenant majoritairement de l'éthylbenzène, un flux comprenant majoritairement du styrène et un flux de composés lourds.

### Définitions

Les composés comprenant du carbone mentionnés dans la description peuvent être d'origine fossile ou biosourcés. Dans ce dernier cas, ils peuvent être, partiellement ou totalement, issus de la biomasse ou obtenus à partir de matières premières renouvelables issues de la biomasse. Sont concernés notamment les polymères, les plastifiants, les charges, etc.

La purification des charges issues de procédés de dépolymérisation de composés styréniques nécessite de prendre en compte les spécificités de ces charges, et en particulier une teneur en composés dits « lourds », c'est-à-dire dont la température d'ébullition est plus élevée que celle du styrène, plus importante que dans les charges issues de procédés de production du styrène par deshydrogénation de l'éthylbenzène, et une teneur en éthylbenzène bien plus faible. Les stratégies de purifications élaborées pour traiter les charges issues de ces procédés de déshydrogénation ne sont donc pas adaptées à la purification des charges issues de procédés de dépolymérisation de composés styréniques.

### Charge du procédé de purification

L'invention a pour objet un procédé de purification d'une charge issue d'un procédé de dépolymérisation de composés styréniques. Par « issue d'un procédé de dépolymérisation de composés styréniques », on entend que la charge est issue d'un procédé produisant du styrène à partir de composés comprenant du styrène, tels que du polystyrène ou des élastomères styréniques. De tels procédés sont connus de l'homme du métier et peuvent être, par exemple, un procédé de pyrolyse, ou un procédé de décomposition enzymatique.

De préférence, la charge du procédé selon l'invention est issue d'un procédé de pyrolyse.

La charge du procédé selon l'invention comprend majoritairement du styrène, c'est-à-dire au moins 50% en poids de styrène, préférentiellement au moins 60% en poids de styrène.

De manière préférée, la charge du procédé de purification comprend au plus 10% poids d'éthylbenzène, de préférence au plus 5% poids d'éthylbenzène et de manière préférée au plus 3% poids d'éthylbenzène.

Préférentiellement, la charge du procédé de purification comprend au moins 10% poids de composés dont le point d'ébullition est supérieur à celui du styrène.

Ces teneurs en éthylbenzène et en composés dits « lourds » distinguent clairement une charge telle que traitée dans le procédé selon l'invention d'une charge issue d'un procédé de production de styrène à partir d'éthylbenzène.

### Étape a) de séparation d'une charge issue d'un procédé de dépolymérisation de composés styréniques

Le procédé selon l'invention comprend une étape de séparation mettant en œuvre une colonne de distillation alimentée en fond de colonne par la charge du procédé de purification et produisant en tête de colonne un extrait riche en composés léger, en fond un raffinat riche en composés lourds, et par un soutirage latérale un flux riche en styrène, ladite colonne ayant pour seul apport de chaleur ladite charge du procédé de purification.

La charge du procédé de purification est à température élevée, de préférence à une température supérieure à 300°C, préférentiellement à une température comprise entre 300°C et 400°C. Cette température est suffisante pour que la colonne ne nécessite pas d'autre apport de chaleur. De manière préférée, le procédé de dépolymérisation de composés styréniques est un procédé de pyrolyse et la charge issue du réacteur de pyrolyse alimente directement le procédé de l'invention, sans opération intermédiaire de refroidissement, chauffe, ou séparation.

En alimentant la charge en fond de colonne, la charge est rapidement refroidie, permettant ainsi de limiter les réactions éventuelles de polymérisation du styrène. Cette alimentation permet en outre une meilleure gestion des composés dits lourds. En effet, l'absence de système de recirculation en fond de colonne, utilisé habituellement pour maintenir en température la colonne à distiller, limite grandement les risques d'encrassement par les composés dits lourds, particulièrement visqueux.

On soutire en fond de colonne un raffinat riche en composés lourds.

En tête de colonne, l'effluent vapeur est refroidi à une température comprise entre 30°C et 50°C, de préférence comprise entre 35°C et 45°C. La fraction liquide condensée est renvoyée en tête de colonne en tant que reflux, tandis que la fraction vapeur est ensuite sous-refroidie à une température comprise entre -5°C et 10°C, préférentiellement entre -5°C et 5°C afin de condenser le styrène éventuellement entrainé avec les composés légers. Le flux condensé après sous-refroidissement est renvoyé en tête de colonne en tant que reflux. La fraction vapeur résiduelle constitue l'extrait riche en composés légers. Cet extrait peut ensuite être valorisé, par exemple sous forme énergétique. Un premier refroidissement permet d'utiliser au maximum de l'eau de refroidissement à température ambiante en tant qu'utilité froide et minimise l'utilisation d'utilité froide spécifique pour obtenir un sous-refroidissement, ce qui impacte favorablement l'analyse de cycle de vie du procédé selon l'invention.

La colonne à distiller mise en œuvre dans l'étape a) du procédé selon l'invention comprend de 5 à 20 étages théoriques, de préférence au plus 15 étages théoriques, de manière préférée de 8 à 12 étages théoriques.

On soutire sur un plateau intermédiaire un flux riche en styrène. Ce plateau de soutirage est situé dans le tiers inférieur de la colonne à distiller, de préférence de 1 à 3 étages théoriques du plateau de fond. Ce soutirage à une position basse de la colonne, légèrement éloigné du plateau de fond, permet de limiter l'entrainement de composés lourds dans le flux riche en styrène et limite d'autant les risques d'encrassement des équipements ultérieurs.

La colonne à distiller mise en œuvre dans l'étape a) du procédé selon l'invention est opérée à une pression comprise entre 0,1 et 2,0 bara, de préférence entre 0,5 et 1,5 bara et de manière préférée entre 0,5 et 1,1 bar, la pression d'opération étant entendue comme la pression mesurée en tête de colonne. Par « bara », on entend bar absolu, par opposition à une pression exprimée en bar relatif, communément noté « barg » selon la notation anglaise « bar gauge ».

L'étape a) du procédé selon l'invention permet de limiter la polymérisation du styrène, avec en particulier un refroidissement rapide de la charge du procédé par l'alimentation de cette charge en fond de colonne. De manière préférée, et afin de limiter encore plus le risque de polymérisation, un inhibiteur de polymérisation du styrène en polystyrène, tel que le 2,2,6,6-tetramethyl-4-oxopiperidinooxy, peut être alimenté dans la colonne à distiller de l'étape a) du procédé, de préférence au niveau de la tête de colonne.

### Étape b) de séparation du flux riche en styrène

Le flux riche en styrène issu de l'étape a) du procédé est séparé dans une étape b) en au moins un flux comprenant majoritairement de l'éthylbenzène, un flux comprenant majoritairement du styrène et un flux de composés lourds.

L'étape b) de séparation permet d'obtenir un flux comprenant majoritairement du styrène pouvant alimenter un procédé de polymérisation du styrène, donc répondant aux spécifications de tels procédés, avec en particulier une teneur en styrène très élevée, de préférence supérieure à 99,5% poids, et des teneurs en composés tels que éthylbenzène, benzène, cumène, alpha-méthyl-styrène et oligomères du styrène très faibles.

Dans un premier arrangement préféré, l'étape b) de séparation du flux riche en styrène comprend deux sections de séparations successives.

Une première section de séparation est alimentée par le flux riche en styrène issu de l'étape a) et permet de séparer un flux comprenant majoritairement de l'éthylbenzène et un raffinat styrénique.

Cette première section est mise en œuvre dans une colonne de distillation comprenant de 60 à 100 étages théoriques, et est opérée à une pression inférieure ou égale à 0,25 bara en tête de colonne de manière à maintenir la température en fond de colonne à une valeur inférieure ou égale à 120°C.

La colonne de distillation de la première section est alimentée par le flux riche en styrène issu de l'étape a) en bas du tiers supérieur de la colonne. Par exemple, pour une colonne comprenant 60 étages théoriques, le flux riche en styrène est alimenté à un étage compris entre le 18^{ème} étage théorique et le 22^{ème} étage théorique, les étages étant numérotés de haut en bas.

Le taux de reflux au condensateur de cette colonne, correspondant au débit massique de reflux alimenté en tête de colonne sur le débit massique de flux comprenant majoritairement de l'éthylbenzène est préférentiellement compris entre 60 et 300. Ce paramètre varie fortement en fonction de la teneur en éthylbenzène du flux riche en styrène. Plus la teneur en éthylbenzène dans le flux riche en styrène sera faible, plus le taux de reflux au condensateur de la colonne sera élevé.

Le taux de reflux au rebouilleur de cette colonne, correspondant au débit massique de reflux alimenté en fond de colonne sur le débit massique de raffinat styrénique est préférentiellement compris entre 4 et 10, de manière préférée compris entre 5 et 9.

Une deuxième section de séparation est alimentée par le raffinat styrénique issu de la première section de séparation et produit un flux comprenant majoritairement du styrène et un flux de composés lourds.

Cette deuxième section est mise en œuvre dans une colonne de distillation comprenant de 40 à 100 étages théoriques, de manière préférée comprenant de 40 à 70 étages théoriques, et est opérée à une pression inférieure ou égale à 0,25 bara en tête de colonne de manière à maintenir la température en fond de colonne à une valeur inférieure ou égale à 120°C.

La colonne de distillation de la première section est alimentée par le raffinat styrénique issu de la première section de séparation dans la partie inférieure de la colonne, préférentiellement dans le haut du cinquième inférieur de la colonne. Par exemple, pour une colonne comprenant 50 étages théoriques, le raffinat styrénique issu de la première section de séparation est alimenté à un étage compris entre le 35^{ème} étage théorique et le 45^{ème} étage théorique, les étages étant numérotés de haut en bas.

Le taux de reflux au condensateur de cette colonne, correspondant au débit massique de reflux alimenté en tête de colonne sur le débit massique de flux comprenant majoritairement du styrène est préférentiellement compris entre 4 et 8.

Le taux de reflux au rebouilleur de cette colonne, correspondant au débit massique de reflux alimenté en fond de colonne sur le débit massique de flux de composés lourds est préférentiellement compris entre 40 et 200, ce ratio étant grandement influencé par la teneur en composés tels que cumène et alpha-méthylstyrène.

Dans un autre arrangement préféré, l'étape b) de séparation du flux riche en styrène est mise en œuvre dans une colonne à distiller à paroi interne.

Une colonne à paroi interne est un équipement de distillation bien connu de l'homme du métier dans lequel une paroi interne étanche aux fluides et disposée verticalement sépare une partie de la colonne en deux zones distinctes. Une colonne à paroi interne est ainsi généralement constituée d'une partie commune inférieure dans laquelle les étages de séparation ne sont pas divisés par la paroi interne, d'une partie divisée dans laquelle les étages de séparation sont divisés par la paroi interne et d'une partie commune supérieure dans laquelle les étages de séparation ne sont pas divisés par la paroi interne.

La colonne à paroi interne comprend au total de 70 à 130 étages théoriques, préférentiellement de 80 à 120 étages théoriques, très préférentiellement de 90 à 110 étages théoriques. La paroi interne est préférentiellement centrée, c'est-à-dire qu'elle partitionne la colonne dans la longueur où elle est présente en deux parties de volume égal. Lorsque le nombre d'étages théoriques de part et d'autre de la paroi interne est différent, par exemple du fait de l'utilisation de types de plateaux distributeurs ou de garnissage différent, le nombre total d'étage théorique de la colonne est entendu comme la somme des étages théoriques des parties communes et du plus grand nombre d'étages entre les deux parties divisées. La colonne est opérée à une pression inférieure ou égale à 0,25 bara en tête de colonne de manière à maintenir la température en fond de colonne à une valeur inférieure ou égale à 120°C.

Le flux riche en styrène issu de l'étape a) du procédé est alimenté d'un côté de la paroi interne sur un étage allant du 10^{ème} au 20^{ème} étage théorique, de préférence allant du 12^{ème} au 18^{ème} étage théorique et très préférentiellement au 15^{ème} étage théorique, les étages étant numérotés de haut en bas.

Dans une première variante de cet arrangement, la partie inférieure commune de la colonne à paroi interne comprend de 8 à 12 étages théoriques et la partie commune supérieure comprend de 8 à 12 étages théoriques. Le flux comprenant majoritairement du styrène est soutiré dans la partie opposée, par rapport à la paroi interne, à la partie où est injectée le flux riche en styrène. Le soutirage est effectué sur un étage proche de la partie supérieure de la partie divisée, préférentiellement sur l'un des 5 étages supérieurs de la partie divisée, de manière préférée sur l'un des 3 étages supérieurs de la partie divisée de manière très préférée sur l'un des deux étages supérieurs de la partie divisée, et très préférentiellement sur le premier étage de la partie divisée, en comptant les étages à partir du haut.

On soutire en tête de colonne le flux comprenant majoritairement de l'éthylbenzène et en fond de colonne le flux de composés lourds.

Le taux de reflux au condensateur de cette colonne, correspondant au débit massique de reflux alimenté en tête de colonne sur le débit massique de flux comprenant majoritairement de l'éthylbenzène est préférentiellement compris entre 60 et 300. Ce paramètre varie fortement en fonction de la teneur en éthylbenzène du flux riche en styrène. Plus la teneur en éthylbenzène dans le flux riche en styrène sera faible, plus le taux de reflux au condensateur de la colonne sera élevé.

Le taux de reflux au rebouilleur de cette colonne, correspondant au débit massique de reflux alimenté en fond de colonne sur le débit massique de flux de composés lourds est préférentiellement compris entre 50 et 200, ce ratio étant grandement influencé par la teneur en composés tels que cumène et alpha-méthylstyrène.

Dans une deuxième variante de cet arrangement, la colonne à paroi interne ne comprend pas de partie commune supérieure. C'est-à-dire que la paroi s'étend jusqu'à la tête de la colonne à paroi interne.

Dans cette variante, la partie commune inférieure comprend de 2 à 12 étages théoriques, préférentiellement de 2 à 10 étages théoriques, et très préférentiellement de 2 à 4 étages théoriques.

Dans cette variante, la colonne à paroi interne comprend préférentiellement au total de 60 à 80 étages théoriques.

Dans cette variante, le flux comprenant majoritairement du styrène est soutiré dans la partie opposée, par rapport à la paroi interne, à la partie où est injectée le flux riche en styrène. Le soutirage est effectué en tête de colonne.

Le flux comprenant majoritairement de l'éthylbenzène est soutiré en tête de colonne dans la même partie que la partie où est injectée le flux riche en styrène.

Le taux de reflux au condensateur de cette colonne pour la partie divisée située du côté de l'alimentation en flux riche en styrène, correspondant au débit massique de reflux alimenté en tête de colonne dans cette partie sur le débit massique de flux comprenant majoritairement de l'éthylbenzène est préférentiellement compris entre 60 et 300. Ce paramètre varie fortement en fonction de la teneur en éthylbenzène du flux riche en styrène. Plus la teneur en éthylbenzène dans le flux riche en styrène sera faible, plus le taux de reflux au condensateur de la colonne sera élevé.

Le taux de reflux au condensateur de cette colonne pour la partie divisée située du côté du soutirage du flux comprenant majoritairement du styrène, correspondant au débit massique de reflux alimenté en tête de colonne sur le débit massique de flux comprenant majoritairement du styrène est préférentiellement compris entre 1 et 10.

Le taux de reflux au rebouilleur de cette colonne, correspondant au débit massique de reflux alimenté en fond de colonne sur le débit massique de flux de composés lourds est préférentiellement compris entre 60 et 200, ce ratio étant grandement influencé par la teneur en composés tels que cumène et alpha-méthylstyrène.

De manière préférée, et afin de limiter encore plus le risque de polymérisation, un inhibiteur de polymérisation du styrène en polystyrène, tel que le 2,2,6,6-tetramethyl-4-oxopiperidinooxy, peut être alimenté dans la, ou les colonnes mises en œuvre dans l'étape b) de séparation, préférentiellement en tête de la, ou des colonnes mises en œuvre dans l'étape b) de séparation.

### Exemples

### Exemple 1

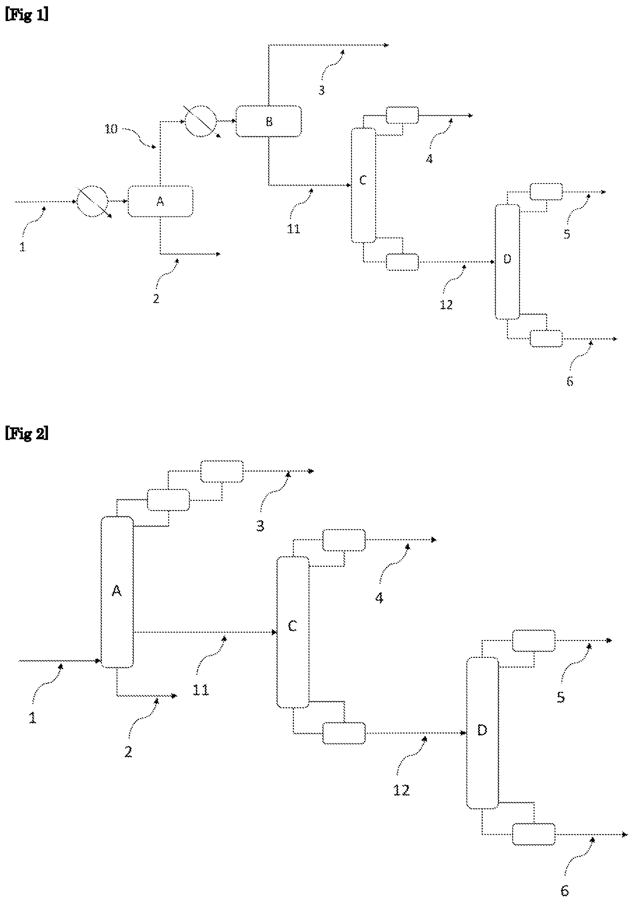

[Fig 1] La figure 1 est une représentation schématique d'un procédé de séparation selon l'art antérieur.

Une charge du procédé de purification (1) alimente, après avoir été refroidie à une température de 209°C, une première étape de séparation (A), opérée à une pression de 1,1 bara, qui sépare cette charge en un raffinat riche en composés lourds (2) soutiré en fond de colonne et une coupe légère (10) soutirée en tête de colonne.

Cette coupe légère (10) est refroidie à une température de 40°C et alimente une deuxième étape de séparation (B), opérée à une pression de 1,1 bara, qui produit en tête un extrait riche en composés légers (3) et en fond un flux riche en styrène (11). Ce dernier alimente une colonne à distiller (C) comprenant, en comptabilisant condensateur et rebouilleur, 60 étages théoriques et opérée à une pression de tête de 0,25 bara, qui produit en tête un flux comprenant majoritairement de l'éthylbenzène (4) et en fond un raffinat styrénique (12). Le flux riche en styrène (11) est alimenté au niveau du 20^{ème} étage théorique en numérotant les étages à partir du condensateur. La température est de 76°C au niveau du condensateur et de 101°C au niveau du rebouilleur. Le taux de reflux en tête de colonne, correspondant au débit de reflux divisé par le débit de flux comprenant majoritairement de l'éthylbenzène (4) (en masse) est égal à 88. Le taux de reflux en fond de colonne, correspondant au débit de reflux divisé par le débit de raffinat styrénique (12) (en masse) est égal à 7.

Le raffinat styrénique (12) alimente une colonne à distiller (D) comprenant, en comptabilisant condensateur et rebouilleur, 50 étages théoriques et opérée à une pression de tête de 0,25 bara, qui produit en tête un flux comprenant majoritairement du styrène (5) et en fond un flux de composés lourds (6). Le raffinat styrénique (12) est alimenté au niveau du 40^{ème} étage théorique en numérotant les étages à partir du condensateur. La température est de 99°C au niveau du condensateur et de 128°C au niveau du rebouilleur. Le taux de reflux en tête de colonne, correspondant au débit de reflux divisé par le débit de flux comprenant majoritairement du styrène (5) (en masse) est égal à 6. Le taux de reflux en fond de colonne, correspondant au débit de reflux divisé par le débit de flux de composés lourds (6) (en masse) est égal à 48.

### Exemple 2

[Fig 2] La figure 2 est une représentation schématique d'un premier arrangement préféré du procédé selon l'invention.

Une charge du procédé de purification (1) alimente en fond de colonne une première colonne à distiller (A) comprenant, en comptabilisant condensateur et rebouilleur, 10 étages théoriques et opérée à une pression de tête de 1 bara, qui sépare cette charge en un raffinat riche en composés lourds (2) soutiré en fond de colonne, un extrait riche en composés légers (3) soutiré en tête et un flux riche en styrène (11) produit par un soutirage latéral, soutiré au niveau du 7^{ème} étage théorique. L'effluent vapeur en tête de colonne est refroidi une première fois à 40°C, la fraction liquide condensée étant renvoyée en tête de colonne, puis sous-refroidi à 2°C afin de condenser le styrène entrainé avec l'effluent vapeur. La fraction liquide condensée après sous-refroidissement est renvoyée en tête de colonne avec la fraction liquide issue du première refroidissement. La fraction vapeur résiduelle constitue l'extrait riche en composés légers (3). La colonne (A) a pour seul apport de chaleur celle apportée par la charge du procédé de purification (1).

Le flux riche en styrène (11) alimente une colonne à distiller (C) comprenant, en comptabilisant condensateur et rebouilleur, 60 étages théoriques et opérée à une pression de tête de 0,25 bara, qui produit en tête un flux comprenant majoritairement de l'éthylbenzène (4) et en fond un raffinat styrénique (12). Le flux riche en styrène (11) est alimenté au niveau du 20^{ème} étage théorique en numérotant les étages à partir du condensateur. La température est de 88°C au niveau du condensateur et de 105°C au niveau du rebouilleur. Le taux de reflux en tête de colonne, correspondant au débit de reflux divisé par le débit de flux comprenant majoritairement de l'éthylbenzène (4) (en masse) est égal à 243. Le taux de reflux en fond de colonne, correspondant au débit de reflux divisé par le débit de raffinat styrénique (12) (en masse) est égal à 7.

Le raffinat styrénique (12) alimente une colonne à distiller (D) comprenant, en comptabilisant condensateur et rebouilleur, 50 étages théoriques et opérée à une pression de tête de 0,25 bara, qui produit en tête un flux comprenant majoritairement du styrène (5) et en fond un flux de composés lourds (6). Le raffinat styrénique (12) est alimenté au niveau du 40^{ème} étage théorique en numérotant les étages à partir du condensateur. La température est de 99°C au niveau du condensateur et de 125°C au niveau du rebouilleur. Le taux de reflux en tête de colonne, correspondant au débit de reflux divisé par le débit de flux comprenant majoritairement du styrène (5) (en masse) est égal à 7. Le taux de reflux en fond de colonne, correspondant au débit de reflux divisé par le débit de flux de composés lourds (6) (en masse) est égal à 89.

### Exemple 3

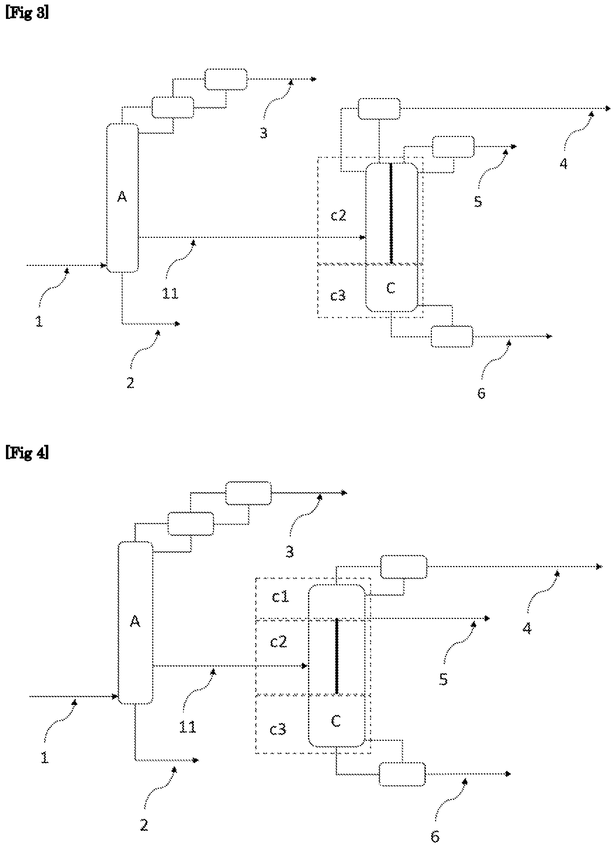

[Fig 3] La figure 3 est une représentation schématique d'une variante d'un deuxième arrangement préféré du procédé selon l'invention.

Une charge du procédé de purification (1) alimente en fond de colonne une première colonne à distiller (A) comprenant, en comptabilisant condensateur et rebouilleur, 10 étages théoriques et opérée à une pression de tête de 1 bara, qui sépare cette charge en un raffinat riche en composés lourds (2) soutiré en fond de colonne, un extrait riche en composés légers (3) soutiré en tête et un flux riche en styrène (11) produit par un soutirage latéral, soutiré au niveau du 7^{ème} étage théorique. L'effluent vapeur en tête de colonne est refroidi une première fois à 40°C, la fraction liquide condensée étant renvoyée en tête de colonne, puis sous-refroidi à 2°C afin de condenser le styrène entrainé avec l'effluent vapeur. La fraction liquide condensée après sous-refroidissement est renvoyée en tête de colonne avec la fraction liquide issue du premier refroidissement. La fraction vapeur résiduelle constitue l'extrait riche en composés légers (3). La colonne (A) a pour seul apport de chaleur celle apportée par la charge du procédé de purification (1).

Le flux riche en styrène (11) alimente une colonne à paroi interne (C) qui produit en tête de la partie où est injectée le flux riche en styrène (11) un flux comprenant majoritairement de l'éthylbenzène (4), et en tête de la partie opposée par rapport à la paroi interne à la partie où est injectée le flux riche en styrène (11) un flux comprenant majoritairement du styrène (5). Cette colonne (C) produit également en fond un flux de composés lourds (6).

La colonne à paroi interne (C) comprend deux zones : une partie divisée (c2) comprenant 67 étages théoriques dans laquelle la paroi interne, s'étendant verticalement, divise la colonne en deux zones entre lesquelles les fluides ne circulent pas, la paroi interne s'étendant jusqu'à la tête de la colonne, et une partie commune inférieure (c3) comprenant 3 étages théoriques. Le flux riche en styrène (11) alimente la colonne à paroi interne (C) au niveau du 20^{ème} étage théorique.

La température du condenseur du côté du soutirage du flux comprenant majoritairement du styrène (5) est de 99°C. Le taux de reflux en tête de colonne pour cette partie, correspondant au débit de reflux divisé par le débit de flux comprenant majoritairement du styrène (5) (en masse) est égal à 6.

La température du condenseur du côté du soutirage du flux comprenant majoritairement de l'éthylbenzène (4) est de 84°C. Le taux de reflux en tête de colonne pour cette partie, correspondant au débit de reflux divisé par le débit de flux comprenant majoritairement de l'éthylbenzène (4) (en masse) est égal à 112.

Le taux de reflux en fond de colonne, correspondant au débit de reflux divisé par le débit de flux de composés lourds (6) (en masse) est égal à 226. La température au niveau du rebouilleur est de 117°C.

On montre dans le tableau 1 les performances de ces différents procédés en termes de consommation d'utilités chaudes (permettant de chauffer les flux), d'utilités froides (permettant de refroidir les flux), de pureté du styrène produit et de rendement en styrène, défini comme le ratio du débit de styrène pur dans le flux comprenant majoritairement du styrène (5) sur le débit de styrène pur dans la charge du procédé de purification (1).

Les consommations en utilités chaude et froide sont exprimées en base 100, sur la base des consommations du procédé illustré par la Figure 1. Une valeur supérieure à 100 témoigne d'une consommation plus élevée.

**[Tableau 1]**

| | **Exemple 1** | **Exemple 2** | **Exemple 3** |
|---|---|---|---|
| Consommation utilité froide | 100 | 102 | 93 |
| Consommation utilité chaude | 100 | 103 | 89 |
| Pureté Styrène | 99.98% | 99.98% | 99.97% |
| Récupération Styrène | 96.8% | 95.5% | 97.1% |

On observe que le procédé de l'exemple 2 permet, avec des performances similaires au procédé de l'exemple 1, de limiter de manière significative les problèmes d'encrassement des échangeurs et des équipements dans les premières étapes de la purification, avec des risques d'arrêt limités et donc une production plus régulière, ce qui a un impact positif sur l'analyse de cycle de vie globale du procédé.

Le procédé de l'exemple 3, outre la limitation des problèmes d'encrassement, permet de plus d'améliorer la récupération de styrène pour une consommation énergétique plus faible, avec un impact encore renforcé sur l'analyse de cycle de vie du procédé.

[Fig 4] La figure 4 est une représentation schématique d'une autre variante d'un deuxième arrangement préféré du procédé selon l'invention.

Une charge du procédé de purification (1) alimente en fond de colonne une première colonne à distiller (A) comprenant, en comptabilisant condensateur et rebouilleur, 10 étages théoriques et opérée à une pression de tête de 1 bara, qui sépare cette charge en un raffinat riche en composés lourds (2) soutiré en fond de colonne, un extrait riche en composés légers (3) soutiré en tête et un flux riche en styrène (11) produit par un soutirage latéral. L'effluent vapeur en tête de colonne est refroidi une première fois à 40°C, la fraction liquide condensée étant renvoyée en tête de colonne, puis sous-refroidi à 2°C afin de condenser le styrène entrainé avec l'effluent vapeur. La fraction liquide condensée après sous-refroidissement est renvoyée en tête de colonne avec la fraction liquide issue du premier refroidissement. La fraction vapeur résiduelle constitue l'extrait riche en composés légers (3). La colonne (A) a pour seul apport de chaleur celle apportée par la charge du procédé de purification (1).

Le flux riche en styrène (11) alimente une colonne à paroi interne (C) qui produit en tête un flux comprenant majoritairement de l'éthylbenzène (4), par un soutirage latéral un flux comprenant majoritairement du styrène (5) et en fond un flux de composés lourds (6).

La colonne à paroi interne (C) comprend trois zones : une partie commune supérieure (c1), une partie divisée (c2) dans laquelle la paroi interne s'étendant verticalement divise la colonne en deux zones entre lesquelles les fluides ne circulent pas, et une partie commune inférieure (c3).

Ainsi, par rapport à la variante illustrée Figure 3, la deuxième colonne (C) comprend une partie commune supérieure (c1).

## Revendications

1. Procédé de purification d'une charge issue d'un procédé de dépolymérisation de composés styréniques, nommée charge du procédé de purification, comprenant au moins les étapes suivantes :
a. Une étape de séparation mettant en œuvre une colonne de distillation alimentée en fond de colonne par la charge du procédé de purification et produisant en tête de colonne un extrait riche en composés léger, en fond un raffinat riche en composés lourds, et par un soutirage latérale un flux riche en styrène, ladite colonne ayant pour seul apport de chaleur ladite charge du procédé de purification ;
b. Une étape de séparation du flux riche en styrène en au moins un flux comprenant majoritairement de l'éthylbenzène, un flux comprenant majoritairement du styrène et un flux de composés lourds.

2. Procédé de purification selon la revendication 1 dans lequel la charge du procédé de purification comprend au plus 10% poids d'éthylbenzène, de préférence au plus 5% poids d'éthylbenzène et de manière préférée au plus 3% poids d'éthylbenzène.

3. Procédé de purification selon l'une quelconque des revendications précédentes dans lequel la charge du procédé de purification comprend au moins 10% poids de composés dont le point d'ébullition est supérieur à celui du styrène.

4. Procédé de purification selon l'une quelconque des revendications précédentes dans lequel la température de la charge du procédé de purification est supérieure à 300°C, préférentiellement comprise entre 300°C et 400°C.

5. Procédé de purification selon l'une quelconque des revendications précédentes dans lequel le procédé de dépolymérisation de composés styréniques est un procédé de pyrolyse et la charge issue du réacteur de pyrolyse alimente directement l'étape a) du procédé de purification, sans opération intermédiaire de refroidissement, chauffe, ou séparation.

6. Procédé de purification selon l'une quelconque des revendications précédentes dans lequel la colonne de distillation de l'étape a) comprend de 5 à 20 étages théoriques, de préférence au plus 15 étages théoriques, de manière préférée de 8 à 12 étages théoriques.

7. Procédé de purification selon l'une quelconque des revendications précédentes dans lequel le condensateur de la colonne de distillation de l'étape a) est opéré à une température comprise entre 30°C et 50°C, l'effluent vapeur de ce condensateur étant ensuite refroidi à une température comprise entre -5°C et 10°C.

8. Procédé de purification selon l'une quelconque des revendications précédentes dans lequel un inhibiteur de polymérisation du styrène est alimenté en tête de colonne dans l'étape a) du procédé.

9. Procédé de purification selon l'une quelconque des revendications précédentes dans lequel l'étape b) de séparation comprend deux sections de séparations successives :
a. Une première section de séparation alimentée par le flux riche en styrène issu de l'étape a) et permettant de séparer un flux comprenant majoritairement de l'éthylbenzène et un raffinat styrénique, mise en œuvre dans une colonne de distillation comprenant de 60 à 100 étages théoriques, et opérée à une pression inférieure ou égale à 0,25 bara en tête de colonne,
b. Une deuxième section de séparation alimentée par le raffinat styrénique issu de la première section de séparation et produisant un flux comprenant majoritairement du styrène et un flux de composés lourds, mise en œuvre dans une colonne de distillation comprenant de 40 à 100 étages théoriques, de manière préférée comprenant de 40 à 70 étages théoriques, et opérée à une pression inférieure ou égale à 0,25 bara en tête de colonne.

10. Procédé de purification selon l'une quelconque des revendications 1 à 8 dans lequel l'étape b) de séparation est mise en œuvre dans une colonne à distiller à paroi interne alimentée par le flux riche en styrène issu de l'étape a) et produisant en tête de colonne un flux comprenant majoritairement de l'éthylbenzène, en fond de colonne un flux de composés lourds et en tant que soutirage latéral, dans une section séparée de la section d'alimentation de la colonne par une paroi interne, un flux comprenant majoritairement du styrène, ladite colonne à paroi interne comprenant de 70 à 130 étages théoriques et étant opérée à une pression inférieure ou égale à 0,25 bara en tête de colonne.

11. Procédé de purification selon la revendication précédente dans lequel la colonne à distiller à paroi interne est constituée d'une partie commune inférieure comprenant de 8 à 12 étages théoriques, d'une partie commune supérieure comprenant de 8 à 12 étages théoriques et d'une partie divisée comprenant le reste des étages théoriques.

12. Procédé de purification selon la revendication 10 dans lequel la colonne à distiller à paroi interne est constituée d'une partie commune inférieure comprenant de 2 à 12 étages théoriques, préférentiellement de 2 à 10 étages théoriques, et très préférentiellement de 2 à 4 étages théoriques, et d'une partie divisée comprenant le reste des étages théoriques.

## Patentansprüche

1. Verfahren zur Reinigung einer Charge, die von einem Depolymerisationsverfahren von Styrolverbindungen stammt, die Charge des Reinigungsverfahrens genannt wird, das zumindest die folgenden Schritte umfasst:
a. einen Schritt zur Trennung, der eine Destillationskolonne einsetzt, die am Kolonnenboden mit der Charge des Reinigungsverfahrens gespeist wird und am Kolonnenkopf ein an leichten Verbindungen reiches Extrakt, am Boden ein an schweren Verbindungen reiches Raffinat und durch eine seitliche Entnahme einen an Styrol reichen Fluss erzeugt, wobei die Kolonne die Charge des Reinigungsverfahrens als einzige Wärmezufuhr aufweist;
b. einen Schritt zur Trennung des an Styrol reichen Flusses in mindestens einen Fluss mit überwiegend Ethylbenzol, einen Fluss mit überwiegend Styrol und einen Fluss von schweren Verbindungen.

2. Reinigungsverfahren nach Anspruch 1, bei dem die Charge des Reinigungsverfahrens höchstens 10 Gewichts-% Ethylbenzol, vorzugsweise höchstens 5 Gewichts-% Ethylbenzol und bevorzugt höchstens 3 Gewichts-% Ethylbenzol umfasst.

3. Reinigungsverfahren nach einem der vorangehenden Ansprüche, bei dem die Charge des Reinigungsverfahrens mindestens 10 Gewichts-% Verbindungen umfasst, deren Siedepunkt höher ist als jener von Styrol.

4. Reinigungsverfahren nach einem der vorangehenden Ansprüche, bei dem die Temperatur der Charge des Reinigungsverfahrens höher ist als 300 °C und vorzugsweise zwischen 300 °C und 400 °C liegt.

5. Reinigungsverfahren nach einem der vorangehenden Ansprüche, bei dem das Depolymerisationsverfahren von Styrolverbindungen ein Pyrolyseverfahren ist und die aus dem Pyrolysereaktor stammende Charge direkt den Schritt a) des Reinigungsverfahrens ohne Zwischenoperation zur Abkühlung, Erwärmung oder Trennung speist.

6. Reinigungsverfahren nach einem der vorangehenden Ansprüche, bei dem die Destillationskolonne des Schritts a) 5 bis 20 theoretische Stufen, vorzugsweise höchstens 15 theoretische Stufen, bevorzugt 8 bis 12 theoretische Stufen umfasst.

7. Reinigungsverfahren nach einem der vorangehenden Ansprüche, bei dem der Kondensator der Destillationskolonne des Schritts a) bei einer Temperatur zwischen 30 °C und 50 °C betrieben wird, wobei der Dampfabstrom dieses Kondensators anschließend auf eine Temperatur zwischen -5 °C und 10 °C abgekühlt wird.

8. Reinigungsverfahren nach einem der vorangehenden Ansprüche, bei dem ein Polymerisationsinhibitor für Styrol am Kolonnenkopf im Schritt a) des Verfahrens eingespeist wird.

9. Reinigungsverfahren nach einem der vorangehenden Ansprüche, bei dem der Schritt b) der Trennung zwei Abschnitte von aufeinander folgenden Trennungen umfasst:
a. einen ersten Trennabschnitt, der mit dem an Styrol reichen Fluss, der von Schritt a) stammt, gespeist wird und ermöglicht, einen Fluss mit überwiegend Ethylbenzol und ein Styrolraffinat zu trennen, der in einer Destillationskolonne mit 60 bis 100 theoretischen Stufen umgesetzt wird und bei einem Druck geringer als oder gleich 0,25 bar absolut am Kolonnenkopf betrieben wird,
b. einen zweiten Trennabschnitt, der mit dem Styrolraffinat gespeist wird, das vom ersten Trennabschnitt stammt, und einen Fluss mit überwiegend Styrol und einen Fluss von schweren Verbindungen erzeugt, der in einer Destillationskolonne mit 40 bis 100 theoretischen Stufen, vorzugsweise mit 40 bis 70 theoretischen Stufen umgesetzt wird und bei einem Druck geringer als oder gleich 0,25 bar absolut am Kolonnenkopf betrieben wird.

10. Reinigungsverfahren nach einem der Ansprüche 1 bis 8, bei dem der Schritt b) der Trennung in einer Destillationskolonne mit Innenwand umgesetzt wird, die mit dem an Styrol reichen Fluss gespeist wird, der von Schritt a) stammt, und am Kolonnenkopf einen Fluss mit überwiegend Ethylbenzol, am Kolonnenboden einen Fluss von schweren Verbindungen und als seitliche Entnahme in einem vom Einspeisungsabschnitt der Kolonne durch eine Innenwand getrennten Abschnitt einen Fluss mit überwiegend Styrol erzeugt, wobei die Kolonne mit Innenwand 70 bis 130 theoretische Stufen umfasst und bei einem Druck geringer als oder gleich 0,25 bar absolut am Kolonnenkopf betrieben wird.

11. Reinigungsverfahren nach dem vorangehenden Anspruch, bei dem die Destillationskolonne mit Innenwand aus einem gemeinsamen unteren Teil mit 8 bis 12 theoretischen Stufen, einem gemeinsamen oberen Teil mit 8 bis 12 theoretischen Stufen und einem unterteilten Teil mit dem Rest der theoretischen Stufen besteht.

12. Reinigungsverfahren nach Anspruch 10, bei dem die Destillationskolonne mit Innenwand aus einem gemeinsamen unteren Teil mit 2 bis 12 theoretischen Stufen, vorzugsweise 2 bis 10 theoretischen Stufen und am meisten bevorzugt 2 bis 4 theoretischen Stufen und einem unterteilten Teil mit dem Rest der theoretischen Stufen besteht.

## Claims

1. Process for the purification of a feedstock resulting from a process for the depolymerization of styrene compounds, referred to as feedstock of the purification process, comprising at least the following steps:
a. a separation step employing a distillation column fed at the column bottom with the feedstock of the purification process and producing, at the column top, an extract rich in light compounds, at the bottom a raffinate rich in heavy compounds and, by a sidestream withdrawal, a stream rich in styrene, said column having as sole heat supply said feedstock of the purification process;
b. a step of separation of the stream rich in styrene into at least a stream comprising predominantly ethylbenzene, a stream comprising predominantly styrene and a stream of heavy compounds.

2. Purification process according to Claim 1, in which the feedstock of the purification process comprises at most 10% by weight of ethylbenzene, preferably at most 5% by weight of ethylbenzene and in a preferred way at most 3% by weight of ethylbenzene.

3. Purification process according to either one of the preceding claims, in which the feedstock of the purification process comprises at least 10% by weight of compounds, the boiling point of which is greater than that of styrene.

4. Purification process according to any one of the preceding claims, in which the temperature of the feedstock of the purification process is greater than 300°C, preferentially of between 300°C and 400°C.

5. Purification process according to any one of the preceding claims, in which the process for the depolymerization of styrene compounds is a pyrolysis process and the feedstock resulting from the pyrolysis reactor directly feeds step a) of the purification process, without intermediate cooling, heating or separation operation.

6. Purification process according to any one of the preceding claims, in which the distillation column of step a) comprises from 5 to 20 theoretical stages, preferably at most 15 theoretical stages, in a preferred way from 8 to 12 theoretical stages.

7. Purification process according to any one of the preceding claims, in which the condenser of the distillation column of step a) is operated at a temperature of between 30°C and 50°C, the vapour effluent from this condenser subsequently being cooled to a temperature of between -5°C and 10°C.

8. Purification process according to any one of the preceding claims, in which an inhibitor of the polymerization of styrene is fed at the column top in step a) of the process.

9. Purification process according to any one of the preceding claims, in which the separation step b) comprises two successive separation sections:
a. a first separation section fed with the stream rich in styrene resulting from step a) and making it possible to separate a stream comprising predominantly ethylbenzene and a styrenic raffinate, carried out in a distillation column comprising from 60 to 100 theoretical stages, and operated at a pressure of less than or equal to 0.25 bara at the column top,
b. a second separation section fed with the styrenic raffinate resulting from the first separation section and producing a stream comprising predominantly styrene and a stream of heavy compounds, carried out in a distillation column comprising from 40 to 100 theoretical stages, preferably comprising from 40 to 70 theoretical stages, and operated at a pressure of less than or equal to 0.25 bara at the column top.

10. Purification process according to any one of Claims 1 to 8, in which the separation step b) is carried out in a divided wall distillation column fed with the stream rich in styrene resulting from step a) and producing, at the column top, a stream comprising predominantly ethylbenzene, at the column bottom a stream of heavy compounds and, as sidestream withdrawal, in a section separated from the feed section of the column by an internal wall, a stream comprising predominantly styrene, said divided wall column comprising from 70 to 130 theoretical stages and being operated at a pressure of less than or equal to 0.25 bara at the column top.

11. Purification process according to the preceding claim, in which the divided wall distillation column consists of a lower common part comprising from 8 to 12 theoretical stages, of an upper common part comprising from 8 to 12 theoretical stages and of a divided part comprising the remainder of the theoretical stages.

12. Purification process according to Claim 10, in which the divided wall distillation column consists of a lower common part comprising from 2 to 12 theoretical stages, preferentially from 2 to 10 theoretical stages and very preferentially from 2 to 4 theoretical stages, and of a divided part comprising the remainder of the theoretical stages.
